# EUROPEAN PATENT APPLICATION

(11) **EP 1 357 132 A2**
(43) Date of publication of application: **29.10.2003**
(21) Application number: 03008916.3
(22) Date of filing: 16.04.2003
(51) Int. Cl.: C07K 16/30, A61K 47/48, G01N 33/574

(54) **Detection of the her2 receptor by means of biotinylated humanized antibody**

(30) Priority: 19.04.2002 IT TO20020340
(71) Applicant: BIOTHER di Contardi Gabriella & C. Società in accomandita semplice, 10123 Torino (IT)
(72) Inventor: Bussolati, Giovanni, 10123 Torino (IT); Sapino, Anna, 10123 Torino (IT)
(74) Representative: Freyria Fava, Cristina

(57) **Abstract**

The present invention involves the localization of HER2 receptor (extra-cellular domain)in breast cancer cells, using a humanized monoclonal antibody. The invention is related to a modification of the Herceptin antibody, so as to maintain the antibody properties, while making the antibody directly recognizable in immunohistochemistry.

## Description

The present invention concerns the *in vitro* localization of HER2 receptor in breast cancer cells.

The HER2 receptor, also known as c-erb-B2, is a member of the epidermal growth factor receptor family. HER2 is a trans-membrane receptor, with an intra-cytoplasmic (internal domain) and an extra-cytoplasmic domain (external domain).

The interest for Her2 is due to the fact that Her2 is present at low level in normal epithelial cells, whereas it is over-expressed in cells of some carcinomas (approximately from 15 to 35% of invasive breast carcinomas over-express HER2 receptor protein). The over-expression of HER2, which induces the appearance of high levels of the antigen over the cell membrane, is the consequence of the amplification of the corresponding gene, being such an amplification detectatble by Southern blot and *in situ* hybridization (FISH and CISH) techniques. FISH (Fluorescence *In Situ* Hybridization) is an *in situ* hybridization technique (ISH), based on the use of probes linked to flourochromes. CISH (Chromogenic *In Situ* Hybridization) is instead based on the use of probes linked either to digoxigenin or to biotin and the reaction is revealed by enzymatic colorimetric methods that have diaminobenzidine as a substrate. Thus, the amplification is demonstrated by the presence of intranuclear precipitates seen by the optical microscope. HER2 gene amplification has been widely studied using both techniques (Tanner et al Am, J Pathol 2000;157:1467-72). In breast carcinomas, HER2 gene amplification has been studied in order to confirm the specificity of immunocytohemical reactions that demonstrate the over-expression of the receptor protein (Ridolfi et al Mod Pathol 2000;13: 866-73).

In addition to breast carcinomas HER2 expression has been studied in tumor of different origin, for example originating from the respiratory tract (Bunn et al Clin Cancer Res 2001; 7:3239-50, Hirashima N et al Mod Pathol 2001; 14: 556-62, Kazkayasi M et al.Eur Arch Otorhinolaryngol 2001; 258:329-35), from the gastrointestinal tract(Ross JS et al Cancer Invest. 2001;19:554-68, Kono K et al Int J Cancer 2002; 98:216-20) and of renal origin (Stumm G et al. Int J Cancer 1996; 69:17-22).

The interest on the localization of HER2 is linked to the recent introduction in the therapy of tumors expressing the receptor protein of a "humanized" antibody, called Trastuzumab or Herceptin® (Genentech, Inc., South San Francisco, CA, License #1048). Herceptin® (distributed in Italy by Roche S.p.A., Milan, national identification number A034949014/E as vials containing 150 mg of powder (Trastuzumab) to be dissolved and administered as endovenous infusion) is a specific antibody directed against the extra-cytoplamic domain of the HER2 receptor (Carter P, et al. Proc Natl Acad Sci U S A. 1992;89:4285-9).

Herceptin® antibody is produced by DNA recombinant genetic technology, wherein the antibody sequence of the antigen binding site of the murine monoclonal antibody 4D5 developed by Genentech Inc. (as described by the US-A-5,677,171 patent) direct against the extra-cellular domain of HER2, is linked to the constant fraction Fc of a human antibody. Thus, Herceptin® antibody works in the same way as a human antibody, being minimal the murine fraction.

The antibody was shown to be effective in the treatment of advanced tumors, but only in cases with over-expression of the HER2 antigen on the cellular membrane, detectable by immunocytochemical techniques (Vogel CL, et al. J Clin Oncol 2002;20:719-26).

Thus, the first priority for eligibility for a therapeutic use of Herceptin® is the demonstration, for example by immunocytochemistry, of the over-expression of the membrane domain of HER2.

At present, different procedures are commercially available for HER2 protein detection.

The USA Food and Drug Administration has validated and approved as immunocytochemical procedure polyclonal and monoclonal antibodies directed against c-erbB2/HER2; among these a rabbit antiserum directed against the aminoacidic sequence of the intra-cytoplasmic domain of HER2 (the polyclonal antibody commercialized as HERCEPTEST by Dako). Widely used is a murine monoclonal antibody (CB11), directed also against the intra-cytoplasmic sequence. Discrepancies on the evaluation of the results on HER2 over-expression are well demonstrated in the literature, depending on test and on antibodies used (Tubbs RR, et al. J Clin Oncol 2002; 19: 2714-21).

During the immunocytochemical reaction, the primary reagent (rabbit antiserum or monoclonal antibody), specific for the target antigen, is followed by a biotinylated secondary antibody that recognizes a constant portion of the primary reagent. Following this reaction, an avidin biotinylated-peroxidase complex links to the secondary antibody allowing the localization of the specific antigen through a peroxidase colorimetric reaction.

It is suggested to report the results of the immunocytochemical reactions of the anti c-erbB2 antibodies using the FDA approved procedure (Birner P, Clin Cancer Res 2001;7:1669-75).

This procedure takes into consideration the membrane localization of the reaction, its intensity (light, moderate, strong) and the percentage of stained cells (higher or lower than 10%). The scoring of these three parameters gives rise to the following semi-quantitative score: 0/1+ (no over-expression), 2+ (light to moderate over-expression) and 3+ (moderate to strong over-expression). Cut-off for the evaluation of positive or negative reaction has been determined on the base of clinical trials evaluating the response to Herceptin® treatment. As a matter of fact among the criteria for patient eligibility to Herceptin® treatment are: (a)the immunocytochemical evidence of the receptor protein over-expression by the evaluation score 3+, or (b)the immunocytochemical evidence of the receptor protein over-expression by the evaluation score 2+ associated to the HER2 receptor gene amplification evaluated by FISH technique (BCNA policy statement, www.bcna.org.au/bcna_policies/herceptin).

The detection of the extra-cytoplasmic portion of the HER2 receptor is thus essential, both in order to obtain valid results with Herceptin® treatment and avoid overtreatment in patients affected by breast carcinoma.

This is stressed by the fact that extra-cellular fragments of the receptor could be either lost or mutated (Mendrola JM, et al. J Biol Chem. 2002;277:4704-12; Siegel PM et al.EMBO J. 1999;18:2149-64).Such receptor alterations would lead to the non-effectiveness of the Herceptin® treatment in patient previously selected on the basis of the positive results with diagnostic tests that recognized the intracytoplasmic portion of the HER2 receptor.

Herceptin® antibody as such cannot be used as primary antibody in immunocytochemical diagnostic tests performed on a human tumor tissue sample - immunocytochemistry - since the secondary antibody directed against human immunoglobulins would react with the serum immunoglobulins present within the tissue, which are non-specific for the extracytoplasmic portion of HER2 receptor, and would cause a false localization.

The present invention, in the preferred embodiment, consists in a modification of Herceptin® antibody, which maintains the antibody reactivity, but allows a direct utilization of Herceptin® as a diagnostic test, for example in immunocytochemistry.

For this purpose the humanized antibody Herceptin® is conjugated with biotin molecules that lind to lateral -NH₂ groups of the Fc portion of the human immunoglobulin present in Herceptin®.

The biotinylated Herceptin® (called BiotHER) can be directly used on tissue sections, where it links to the extra-cytoplamic portion of HER2 forming the antigen-antibody complex. Biotin will be then revealed owing to the specific finding of the protein with avidin or streptavidin conjugated with a marker, such as an enzyme (e.g. horse radish peroxidase), a radioactive, fluorescent or chemiluminiscent substance.

As an alternative, the humanized antibody Herceptin®, instead of being directly marked with biotin, can be linked in vitro with an anti-human IgG antibody or a corresponding fragment, for example a FAB fragment marked with biotin.

The specific receptor-ligand system constituted by biotin-avidin or biotin-streptavidin can be substituted by any other receptor-ligand system that allows to detect the primary antibody by exploiting the binding affinity that the ligand conjugated to the antibody has for its receptor. Examples of receptor-ligand complex are fluorescin isothiocyanate-anti-fluorescin antibodies and digoxygenin-anti digoxygenin antibodies.

The *in vitro* diagnostic test here described shows definite advantages as compared to the methods used so far. First of all, it recognizes the same antigenic site (extra-cytoplasmic) that is responsible of the therapeutic function of Herceptin® *in vivo.* This point is very important because, as previously said, a shedding of HER2 extra-cytoplasmic fragment in the serum of patient with breast carcinoma may happen (Molina R et al. Anticancer Res. 1999;19:2551-5), with preservation of the intra-cytoplasmic fragment. In these cases, immunocytochemical tests such as HERCEPEST (Dako) that employ antibodies directed against the HER2 intra-cytoplasmic domain will give positive results, while the Herceptin® treatment will be ineffective.

As a second point the procedure is much faster and less prone to mistakes because the proposed procedure is one step shorter, (biotinylated antibody + avidin-peroxidase) instead of (primary antibody + biotinylated secondary antibody + avidin-peroxidase). The procedure is faster (about 1-2 hours less) because the incubation of the sections with the secondary antibody and the corresponding washing steps are avoided; it is less expensive because one reagent is avoided and in addition it avoids the use of monoclonal antibodies and polyclonal sera that might give rise to unspecific reactions. In fact, the unmique antibody applied is the one with definite reactivity, applied and validated for therapy.

In addition, this method gives a specific staining of the target cells (with absolutely negative staining of other cellular types) and exclusive membrane reactivity. On the contrary, using other commercially available antibodies reactivity of normal epithelial cells and a diffuse cytoplasmic staining are sometimes observed. This leads to some difficulties in the interpretation of results concerning both specificity of the reaction and the scoring of the membrane staining, if any.

The cytoplasmic reaction gives rise to important interpretation problems, but consensus in the literature indicates that this localization is an artifact (Taylor et al Oncol Res 1999; 11:311-7). Thus, guidelines on the interpretation of HER2 staining in immunocytochemistry suggest to consider as invalid the reaction when the cytoplasm is stained and to disregard it (American Pathologist Consensus Statement 1999. Arch Pathol Lab Med 2000, 124:966)

The use of the humanized biotinylated antibody BiotHER in immunohistochemistry gives definite advantages as compared to the use of the 4D5 murine monoclonal antibody. It is important to remind that the antibody Herceptin® has been obtained - with DNA recombinant technology - keeping the sequence of the binding site of 4D5 murine antibody directed against the HER2 extra-cytoplasmic domain. The comparison in a series of mammary carcinomas stained on serial sections with either the BiotHER procedure and with the 4D5 monoclonal antibody has demonstrated similar results. However, the samples stained with 4D5 antibody show often a diffuse cytoplasmic staining, that disturbs the reading of the samples and might lead to mistakes in the diagnosis. So far Herceptin® treatment was given only to patients whit high immunocytochemical expression (score 3+) of HER2. It is possible that using for diagnostic purposes the same antibody (biotinylated Herceptin® - BiotHER) used for treatment (Herceptin®)even cases with score 1+ or 2+ of the immunocytichemical reaction could be selected and potentially benefit of Herceptin® treatment as well.

Recently, clinical trials have been performed using humanized antibodies direct against another member of epidermal growth factor family, specifically against the "epidermal growth factor receptor" (EGFr) using the humanized monoclonal antibody EMD72000, in patients with laringeal and hypolaringeal carcinomas (Bier H et al Cancer Chemother Pharmacol 2001; 47: 519-24). Specific EGFr antibodies have been used for treatment of tumor of non epithelial origin as cerebral gliomas (Kuan CT Brain Tumor Pathol 2000; 17: 71-8). The diagnostic application of this antibody or of antibodies against other members of the epidermal growth factors family using the biotinylation procedure herein described might have an important clinical impact, because in analogy to BiotHER application, it might allow to specifically and directly recognize tumors that would benefit of treatment with humanized antibodies.

### Detailed description of embodiments of the present invention

The invention will be now described in detail, purely by way of non-limitative examples, referring to the following drawings, wherein:
- Figure 1 shows a section of a breast carcinoma immunocytochemically stained using BiotHER. On the left of the figure cells invasive breast carcinoma show to over-express HER2 as demonstrated by the intense membrane staining (black staining), whereas on the right a normal duct of the mammary gland does not express HER2 and does not show any cytoplasmic or membrane staining. Nuclei counter-stained with Hematoxilin;
- Figure 2 shows a section of breast carcinoma immunocytochemically stained using BiotHER. In this case 100% of cells show an intense staining that is localized only to the membrane (score 3+);
- Figure 3 shows a section of breast carcinoma immunocytochemically stained using BiotHER. In this case 80% of cells shows a moderate staining that is localized only to the membrane (score 2+);
- Figure 4 shows a section of a cluster of breast carcinoma cells obtained after centrifugation of a metastic pleural effusion. The immunocytochemical reaction using BiotHER shows the strong (score3+) membrane over-expression of HER2.
- Figures 5 and 6 refer to the same area of breast carcinoma stained, either with the murine monoclonal antibody 4D5 ( figure 5) and with BiotHER antibody (figure 6).

### Example 1

Herceptin® (Trastuzumab) produced by Roche is commercially available, distributed in vials and used for breast carcinoma therapy. One mg of immunoglobulin is obtained from a vial of Herceptin® and diluted in physiologic saline solution at a concentration of 1 mg/ml and then dialyzed overnight against a 0.1M solution of Na₂CO₃(sodium bicarbonate) at pH 8.5.

To 1 ml of solution containing 1 mg of Herceptin®, 0.12 ml of ε- caproylamido-biotin-n-hydroxy-succinimide ester (Biospa) is added. The preparation is well mixed for 4 hours at room temperature. Finally, the preparation is dyalized against buffered physiologic solution at pH 7.2 (PBS, physiologic solution buffered with NaH₂PO₄ and Na₂HPO₄ 0.1M).

The final solution (called BiotHER- mother solution) is diluted 1:10 in PBS containing Sodium Azyde 0.1% and then stocked in a refrigerator at 4°C. the reactive is stable for months

The test is performed on breast carcinoma sections (routinely fixed in formaldheyde 4% and paraffin embedded) or on secondary lesions of breast carcinoma (lymph node metastases, carcinoma cells in pleural effusion). *In vitro* cultured cells of a stabilized breast carcinoma cell line BT474, over-expressing HER2 are used as positive control. BT474 cells fixed in 4% formaldheyde are processed following routine cytology procedures in order to obtain a cell pellet to be embedded in paraffin. Test and control sections are then deparaffinized and brought to water.

The sections are covered with a drop of biotinylated Herceptin® (BiotHER), final dilution 1:200 in PBS .

The sections are incubated for 60 minutes at room temperature. The sections are then washed twice for 5 minutes in PBS-Tween and incubated in streptavidin-peroxidase conjugate diluted 1:50 (Biogenex) for 13 minutes at room temperature. After this, the sections are washed in H₂O and then the reaction is developed in a solution of 3'-3-diaminobenzidine (DAB) and H₂O₂ for 5 minutes. After PBS washing the nuclei are counter stained with Mayer Haemalum for 30 seconds, then dehydrated in alcohols, clarified in xilol. The slides are then mounted for microscopic examination with a coverslip using as a mounting medium 1 drop of Entellan.

Positive reactions correspond to the membrane staining.

### Example 2

A series of 47 breast carcinomas has been studied with BiotHER. In all cases the amplification of HER2 gene has been evaluated using the CISH procedure. All cases have been studied on parallel sections using the immunocytochemical procedure and the following antibodies: Herceptest (Dako), CB11 (Ylem, diluted 1:80 incubated for 30 minutes at 37° after microwave treatment), TAB250 (Zymed, diluted 1:40 incubated for 30 minutes at 37°). For CB11, a pre-treatment for the sections for antigen-retrieval was performed by immersion in 0.01 mol/L citrate buffer, pH 7.3, at 94°C for 20 minutes, using a temperature-controlled microwave oven. After blocking non-specific binding sites (using the blocking reagent Histostain Plus kit, Zymed) the sections have been incubated with the specific primary antibody (Herceptest, or CB11, or TAB250). After incubation with the primary antibody the slides have been incubated for 20 minutes with the secondary biotinylated antibody and then with peroxidase-cojugated streptavidin (1/50, StrAviGen MultiLink® Kit, BioGenex) and then for additional 20 minutes at room temperature. Finally, a chromogenic solution of di 3'-3-diaminobenzidine (DAB) (LiquidDAB Substrate Pack, BioGenex) has been used to reveal the reaction. The slides have been counterstained in Mayer Haematoxylin (Biogenex) for 30 seconds, dehydrated and mounted in balsam.

On this series the BiotHER procedure has been applied as previously described (see example 1).

The evaluation of immunocytochemically stained samples has been performed using the scoring system approved by FDA for Herceptest. Briefly, only the cytoplasmic membrane reaction has been considered as valid and the score evaluation has been done considering the percentage of positive cells, the intensity of reaction and the completeness of the membrane staining.

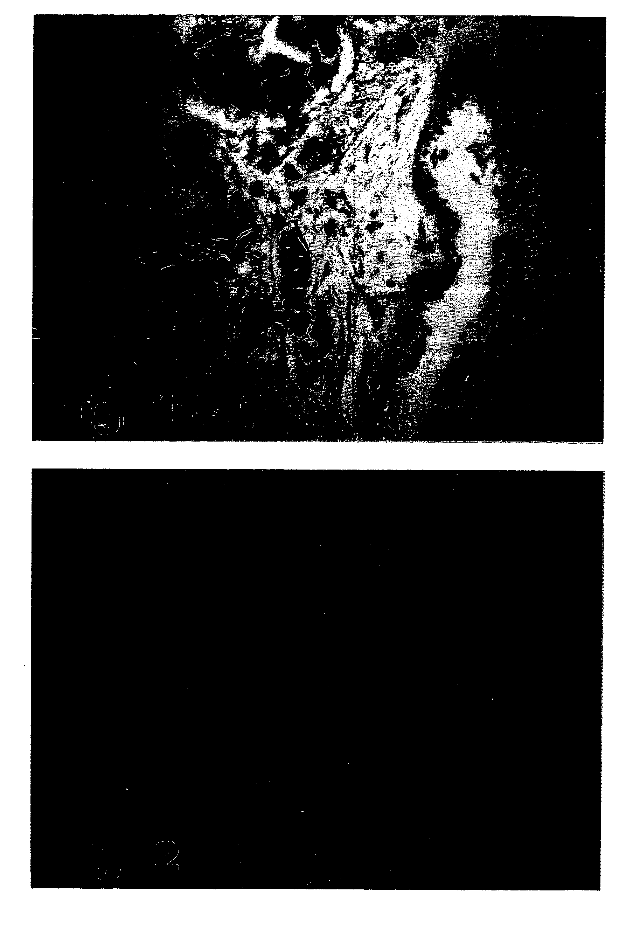

Table 1 shows the results. None of the cases stained with BiotHER showed staining of normal epithelial cells of ducts or lobules, nor staining of stromal or inflammatory cells. Only the carcinoma cells showed a membrane staining and did not show any background staining of the background or of the cytoplasm (figure 1).

**TAB 1:**

| Comparative evaluation of immunocytochemical results using different anti c-erbB2 antibodies and BiotHER | | | | | |
|---|---|---|---|---|---|
| 12 cases with AMPLIFIED HER2 (CISH) | | | | | |
| CB11 | | HERCEPTEST | | BiotHER | |
| score | case n° | score | case n° | score | case n° |
| 0/1+ | 0 | 0/1+ | 1 | 0/1+ | 3 |
| 2+ | 5 | 2+ | 3 | 2+ | 3 |
| 3+ | 7 | 3+ | 8 | 3+ | 6 |

| 35 cases with NOT AMPLIFIED HER2 (CISH) | | | | | |
|---|---|---|---|---|---|
| CB11 | | HERCEPTEST | | BiotHER | |
| score | case n° | score | case n° | score | case n° |
| 0/1+ | 25 | 0/1+ | 27 | 0/1+ | 35 |
| 2+ | 6 | 2+ | 7 | 2+ | 0 |
| 3+ | 4 | 3+ | 1 | 3+ | 0 |

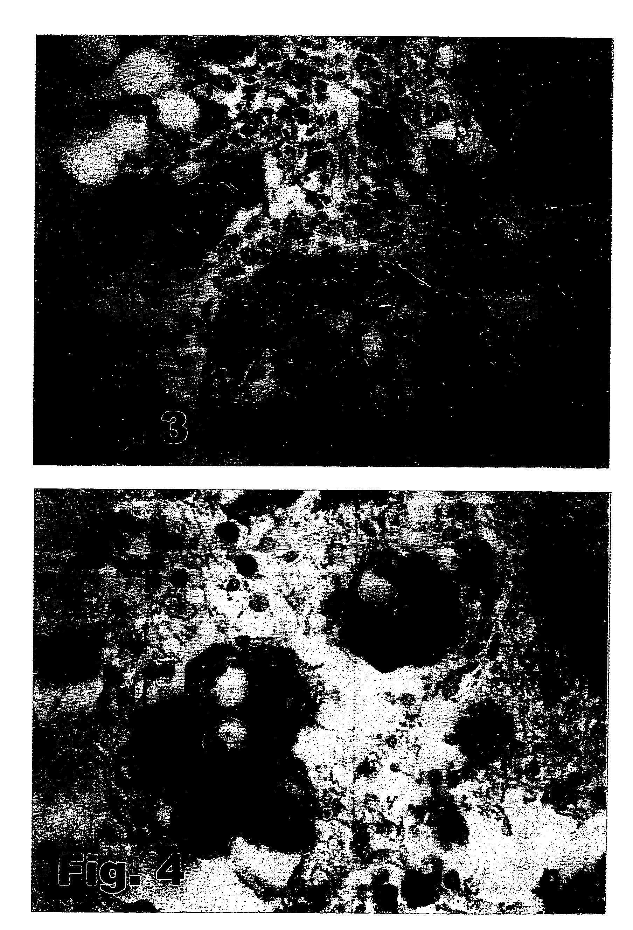

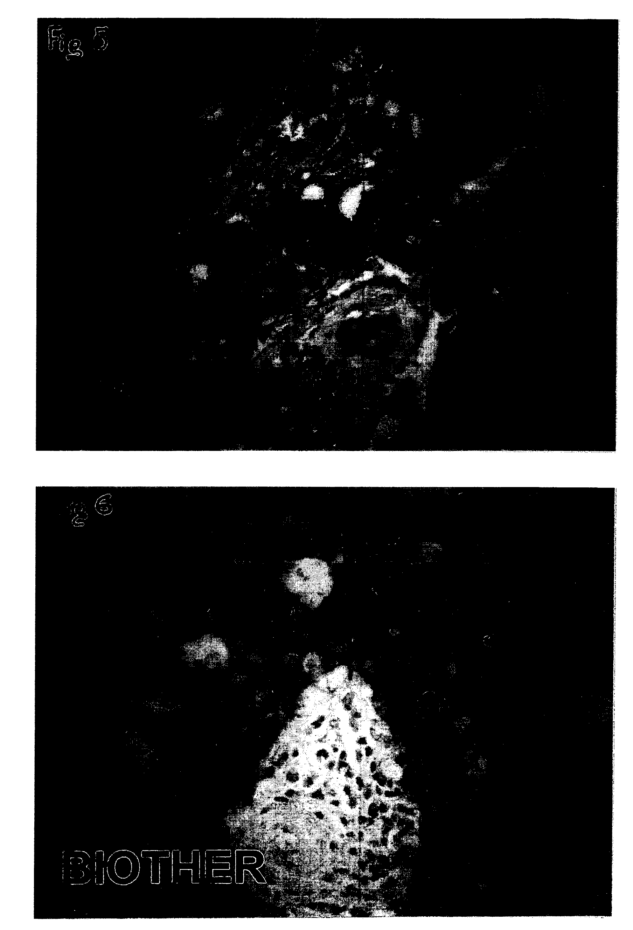

Data of table 1 show that BiotHER test is positive in the majority (about 85%) of breast carcinomas with amplified HER2 gene. Specifically, BiotHER recognized high expression (score 3+) (figure 2) of the extra-cytoplasmic domain of the receptor target of Herceptin® in 50% of cases showing HER2 gene amplification. A lower reactivity (score 2+) (figure 3),but still helpful to demonstrate that Herceptin® can react even in a lower percentage of cells, was demonstrated in 25% of carcinomas with gene amplification. In HER2 non-amplified cases BiotHER was never reacting, demonstrating the high specificity of the reaction.

Thus, it can be argued that BiotHER allows recognizing in a highly specific way about 85% of cases of breast carcinomas with HER2 gene amplification that will completely or partially respond to Herceptin® treatment.

Table 2 shows the clinical response to Herceptin® on recurrences of different patients selected following a score 3+ of Herceptest (Dako).

**TAB 2.**

| Evaluation of the answer to Herceptin® depending on the score obtained with different antibodies and BiotHER | | | | | |
|---|---|---|---|---|---|
| Diagnosis | Score CB11 | Score Herceptest | Score BiotHER | CISH | Time of response (months) |
| Metastatic pleural effusion | 3+ | 3+ | 3+ | HER2 amplif | 18 |
| Metastatic pleural effusion | 3+ | 3+ | 0 | HER2 amplif | 6 |
| Metastatic pleural effusion | 3+ | 3+ | 0 | HER2 amplif | 9 |
| Metastatic pleural effusion | 3+ | 3+ | 0 | HER2 amplif | 3 |
| Sovra-clavear Metastic lymph node | 2+ | 3+ | 3+ | HER2 amplif | 15 |

As shown in Table 2, independently from HER2 gene amplification, the duration time of answer to Herceptin® is reduced in patients whose tumor is not reacting with BiotHER (score 0/1+), as compared to that of patients (case n°1 and 5) with an expression score 3+ (figure 4). This result further confirms the high specificity of BiotHER in recognizing cases that would benefit of Herceptin® treatment as suggested in Table 1.

### Example 3

Sections of breast carcinoma (see above) were dewaxed, brought to water and treated with a reagent formed of Herceptin® conjugated with monovalent biotinylated FAB fragments of anti-human IgG antibodies.

The procedure to prepare the reagent is as follows:the Herceptin® antibody, at a concentration of 1mg/ml is mixed in vitro with biotinylated monovalent FAB fragments of anti-human IgG rabbit antibodies (acquired from commerce from LISTAR, Milan) at a concentration of 1 mg/ml. Herceptin® and FAB fragments were mixed in a 1:1 ratio, so that the ratio was 1 molecule of Herceptin® to 4 molecules of FAB fragments. The mixture is incubated at 37°C for 30 min, then applied on the sections instead of BiotHER.

The principle of this procedure is that the humanized antibody Herceptin® is bound to an excessof biotinylated specific antibody fragments. The FAB fragments, being monovalent, do not produce a precipitation of the antibody and they bind only to the "human" part of Herceptin®, while the antibody site, murine, is not bound.

Sections are incubated with the above complex for one hour, then washed and treated as in the above exemples, with avidin-biotinylated peroxidase complexes, in order to reveal the biotin bound to the FAB fragments, hence to the humanized Herceptin® antibody.

The results obtained with this procedure in a case series of 20 breast carcinomas were similar to those obtained with the above examples using biotinilated Herceptin®.

### Example 4

In a series of 20 cases of breast carcinoma, already examined with the immunocytochemical procedures as above, we made serial parallel sections. One of these sections was stained with the BiotHER procedure as above described, while other section wAS treated with the murine 4D5 monoclonal antibody, directed against the same antigenic site recognized by Herceptin®.

The murine 4D5 antibody was used at a concentration of 1 microgram/ml, similar to that currently used for other murine antibodies, such as TAB 250 and CB11 antibodies. Following the incubation with the 4D5 antibody, the sections were incubated for 20 min with the secondary biotinylated antibody and finally with the streptavidin-peroxidase complexes (1/50, StrAviGen MultiLink® Kit, BioGenex) for additional 20 minutes at room temperature. Finally, a chromogenic solution of 3'-3-diaminobenzidine (DAB) (LiquidDAB Substrate Pack, BioGenex) was employed to reveal the reactions. Slides were counterstained with Mayer's Haematoxylin (Biogenex) for 30 seconds, dehydrated and mounted in balsam.

The comparison, on serial sections, of the same cases and tumor areas stained either with the BiotHER procedure or with the 4D5 antibody showed analogies, being the same cases and areas positive or negative. However, we observed that while staining with BiotHER is only at the cell membrane, staining with 4D5 gives, in addition to the membrane staining, also a diffuse cytoplasmic staining, similar to that observed when using other murine or rabbit anti-HER2 receptor antibodies. It is well known that such cytoplasmic staining can cause a severe disturbance of the result interpretation and that it can lead to erroneous diagnoses.

### Example 5

In addition to the above described in vitro procedures, Biotinylated Herceptin ( BiotHER) can be used for the in vivo imaging and treatment of HER2 positive tumors, using a technique with avidin-radioactive biotin, similar in principle to the above described immunocytochemical procedure.

Herceptin is biotinylated following the procedure described in detain in Example 1.

The reagent is then injected in patients with breast cancer where over-expression of HER2 receptor antigen was previously demonstrated by immunocytochemical procedures ( Example 2).

To the patients, BiotHER is administered endovenously at a concentration of 35 mg/m2 ( 1^{st} step). Thirtysix hours later, 30 mg of avidin and 50 mg of streptavidin are injected. Biotin labeled with Y⁹⁰ or In¹¹¹ is injected endovenously 18-24 later.

This procedure is similar in principle to that used for the diagnosis and therapy of human gliomas expressing tenascin (Paganelli et al. Eur. J. Nucl. Med. 26:348-57, 1999). However, in the latter procedure murine anti-tenascin antibodies are used in the first step. Use of murine antibodies gives rise to problems involved in the liver and spleen binding, which are avoided when using the humainized Herceptin antibody.

The advantage for the diagnosis and treatment of HER2 over-expressing carcinomas is linked to the use of the humanized antibody avoiding the binding to extra-tumoral sites, mainly to liver and spleen, of murine antibodies. The FC fragment of the latter is in fact recognized as heterogeneous by the lympho-reticular tissue of the patients.

## Claims

1. Humanized monoclonal antibody specific for the extra-cytoplasmic domain of HER2 receptor conjugated to either one of the receptor or the ligand of a receptor-ligand system.

2. Antibody according to claim 1, **characterized in that** the humanized monoclonal antibody is Herceptin®.

3. Antibody according to claim 1 or claim 2, **characterized in that** said receptor-ligand system is the biotin-avidin system or the biotin-streptavidin system.

4. Antibody according to any of the claims 1 to 3, **characterized in that** said antibody is conjugated to biotin.

5. Antibody according to any of the claims 1 to 3, **characterized in that** said antibody is conjugated to FAB fragments of anti-human IgG antibodies conjugated to biotin.

6. Method for detecting the extra-cytoplasmic domain of HER2 receptor comprising the following steps:
a) making available a humanized monoclonal antibody specific for the extra-cytoplasmic domain of HER2 receptor;
b) incubating a cell sample with said antibody, in order to allow the formation of an antigen-antibody complex;
c)detecting said antigen-antibody complex.

7. Method according to claim 6, **characterized in that** it includes the step of using as said humanized monoclonal antibody Herceptin®.

8. Method according to claim 6 or claim 7, **characterized in that** it includes the step of using, as said antibody, an antibody conjugated to either one of the receptor or the ligand of a receptor-ligand system.

9. Method according to claim 6 or claim 7, **characterized in that** it includes the step of using, as said antibody, an antibody conjugated to FAB fragments of anti-human IgG antibodies conjugated to either one of the receptor or the ligand of a receptor-ligand system.

10. Method according to claim 8 or claim 9, **characterized in that** it includes the step of reacting said antigen-antibody complex with the other of the ligand and the receptor of said receptor-ligand complex conjugated to a marker, said reaction allowing the detection of said antigen-antibody complex.

11. Method according to claim 10, **characterized in that** it includes the step of using as said marker an enzyme, a radio-active, a fluorescent or a chemiluminescent substance.

12. Method according to any of the claims 8 to 11, **characterized in that** it includes the step of using as said receptor-ligand system the avidin-biotin or streptavidin-biotin system.

13. Method according to any of the claims 6 to 12, **characterized in that** said cell sample is constituted by tumor cells of breast, lung, colonic, renal or gastric tumors.

14. Kit for detecting the extra-cellular domain of the HER2 receptor, comprising an antibody according to any of the claims 1 to 5 into an appropriate vial.

15. Pharmaceutical composition with appropriate vials containing:
a) an antibody according to claims 1,2,4 or 5;
b) avidin or streptavidin
c) biotin conjugated to a radioactive substance.

16. Composition according to claim 15, **characterized in that** biotin is conjugated to Y⁹⁰ or In¹¹¹

17. Method for detecting receptors belonging to the Epidermal Growth Factor family, such as EGFr, and specifically of the extra-cytoplasmic domain of EGFr, comprising the steps of:
a) making available a humanized or human antibody, specific for the extra-cytoplasmic domain of the EGFr receptor;
b) incubating a cell sample with said antibody, in order to allow the formation of an antigen-antibody complex
c) detecting said antigen-antibody complex.
